# EUROPEAN PATENT SPECIFICATION

(11) **EP 3 195 808 B1**
(45) Date of publication and mention of the grant of the patent: **28.11.2018**
(21) Application number: 15834482.0
(22) Date of filing: 09.03.2015
(51) Int. Cl.: A61B 8/12, B06B 1/06

(54) **ULTRASOUND ENDOSCOPE AND ULTRASOUND OBSERVATION DEVICE**
ULTRASCHALLENDOSKOP UND ULTRASCHALLBEOBACHTUNGSVORRICHTUNG
ENDOSCOPE À ULTRASONS ET DISPOSITIF D'OBSERVATION À ULTRASONS

(30) Priority: 18.08.2014 JP 2014166002
(43) Date of publication of application: 26.07.2017
(73) Proprietor: Olympus Corporation, Tokyo 192-8507 (JP)
(72) Inventor: HATAKEYAMA, Tomoyuki, Tokyo 192-8507 (JP); KODAMA, Hiroshi, Tokyo 192-8507 (JP); OKUNO, Yoshiyuki, Tokyo 192-8507 (JP); YOSHIDA, Satoshi, Tokyo 192-8507 (JP)
(74) Representative: Schicker, Silvia
(86) International application number: PCT/JP2015/056777
(87) International publication number: WO 2016/027486

(56) References cited:
- JP-A- 2013 027 695

## Description

### Technical Field

The present invention relates to an ultrasound endoscope which contains an ultrasound transducer in a housing provided at a distal end portion of an insertion portion to perform observation by ultrasound, and an ultrasound observation apparatus.

### Background Art

Conventionally, an ultrasound endoscope provided with an ultrasound transmitting/receiving portion at a distal end portion of an elongated endoscope insertion portion and configured to acquire an ultrasonogram of an inside of a subject by transmitting/receiving ultrasound with the subject as a target has been widely used in a medical field and the like. Recently, downsizing, higher resolution and higher functionality have been required for such an ultrasound endoscope also. For example, in Japanese Patent Application Laid-Open Publication No. 2007-307289, an acoustic lens including a first lens portion covering a first ultrasound transducer and a second lens portion covering a second ultrasound transducer is provided. A technique of an ultrasound endoscope is disclosed in which the second lens portion is provided adjoining a treatment instrument guiding-out portion and is formed thicker than the first lens portion so as to be in contact with a side portion of a treatment instrument when the treatment instrument is guided out from the treatment instrument guiding-out portion, in order that a puncture treatment instrument is certainly detected.

In a case of realizing the ultrasound endoscope as disclosed in Japanese Patent Application Laid-Open Publication No. 2007-307289 as described above, strict assembly accuracy is required so that functions of the ultrasound endoscope can be exerted sufficiently and accurately. Similarly, there is a problem that a higher technique is required for assembly work in order to realize downsizing and higher resolution accurately for an ultrasound endoscope.

A closest prior art endoscope can be considered to be disclosed by JP 2013/27695. The closest prior art is formed by an ultrasound endoscope comprising
- a housing in which an opening portion is formed;
- an ultrasound transmitting/receiving portion comprising an ultrasound transducer group comprising at least one ultrasound transducer and causing a transmission wave to be emitted from the opening portion; and
- wiring connecting to each of ultrasound transducers constituting the ultrasound transducer group.

The present invention has been made in view of the above situation, and an object is to provide an ultrasound endoscope capable of easily and accurately realizing downsizing, higher resolution and higher functionality without necessity of careful positioning, formation of a fitting portion and the like, and an ultrasound observation apparatus.

### Disclosure of Invention

### Means for Solving the Problem

An ultrasound endoscope according to an aspect of the present invention is defined in claim 1.

Further, an ultrasound observation apparatus according to an aspect of the present invention is defined in claim 3.

### Brief Description of the Drawings

Fig. 1 is a diagram illustrating a configuration of an ultrasound observation apparatus according to a first embodiment of the present invention;
Fig. 2 is a cross-sectional view of an ultrasound transmitting/receiving portion and a housing of an ultrasound endoscope according to the first embodiment of the present invention;
Fig. 3 is a cross-sectional view showing a state that an acoustic lens portion is removed from the ultrasound transmitting/receiving portion of the ultrasound endoscope according to the first embodiment of the present invention;
Fig. 4 is an enlarged view of an A portion in Fig. 2;
Fig. 5 is a cross-sectional view of an ultrasound transmitting/receiving portion and a housing of an ultrasound endoscope according to a second embodiment of the present invention;
Fig. 6 is a cross-sectional view showing a state that an acoustic lens portion and protecting portions are removed from the ultrasound transmitting/receiving portion of the ultrasound endoscope according to the second embodiment of the present invention;
Fig. 7 is an enlarged view of the A portion in Fig. 2; and
Fig. 8 is a functional block diagram of an ultrasound observation control apparatus according to a third embodiment of the present invention.

### Best Mode for Carrying Out the Invention

A first embodiment of the present invention will be described below based on drawings.

### (First Embodiment)

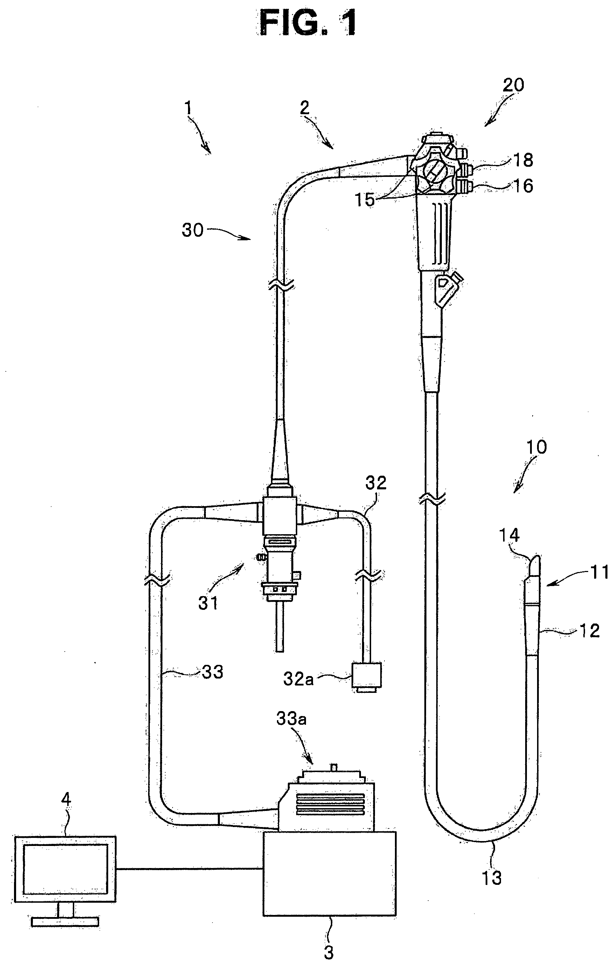

Figs. 1 to 4 show the first embodiment of the present invention. In Fig. 1, reference numeral 1 denotes an ultrasound observation apparatus. Schematically, the ultrasound observation apparatus 1 is an apparatus for obtaining an ultrasonogram (B-mode image) of a predetermined site in a subject by electronically scanning an inside of the subject with ultrasound beam.

The ultrasound observation apparatus 1 has a form of a so-called ultrasound endoscope configured to perform ultrasound beam scanning in a subject. Note that the form of the ultrasound observation apparatus 1 is not limited to the ultrasound endoscope but may be a form of an ultrasound probe configured to be introduced into a subject via a conduit of an endoscope or a form of performing ultrasound beam scanning toward an inside of a subject from outside of the subject.

The ultrasound observation apparatus 1 is configured mainly with an ultrasound endoscope 2, an ultrasound observation control apparatus 3 and a monitor 4.

The ultrasound endoscope 2 is configured having an elongated insertion portion 10 configured to be inserted into a body, an operation portion 20 connectedly arranged at a proximal end of the insertion portion 10, and a universal cord 30 extending from a side portion of the operation portion 20.

A proximal end portion of the universal cord 30 is provided with a connector 31 to which a light source apparatus (not shown) is connected. From the connector 31, a cable 32 connected to a camera control unit (not shown) via a connector 32a and an ultrasound cable 33 attachably and detachably connected to the ultrasound observation control apparatus 3 via a connector 33a are extended. To the ultrasound endoscope 2, the ultrasound observation control apparatus 3 is connected via the connector 33a, and, furthermore, the monitor 4 is connected via the ultrasound observation control apparatus 3.

The insertion portion 10 of the ultrasound endoscope 2 is configured mainly with a distal end rigid portion 11, a bending portion 12 positioned at a rear end of the distal end rigid portion 11, and a long flexible tube portion 13 with a small diameter and flexibility positioned at a rear end of the bending portion 12 and leading to the operation portion 20, which are connectedly arranged from a distal end side in that order.

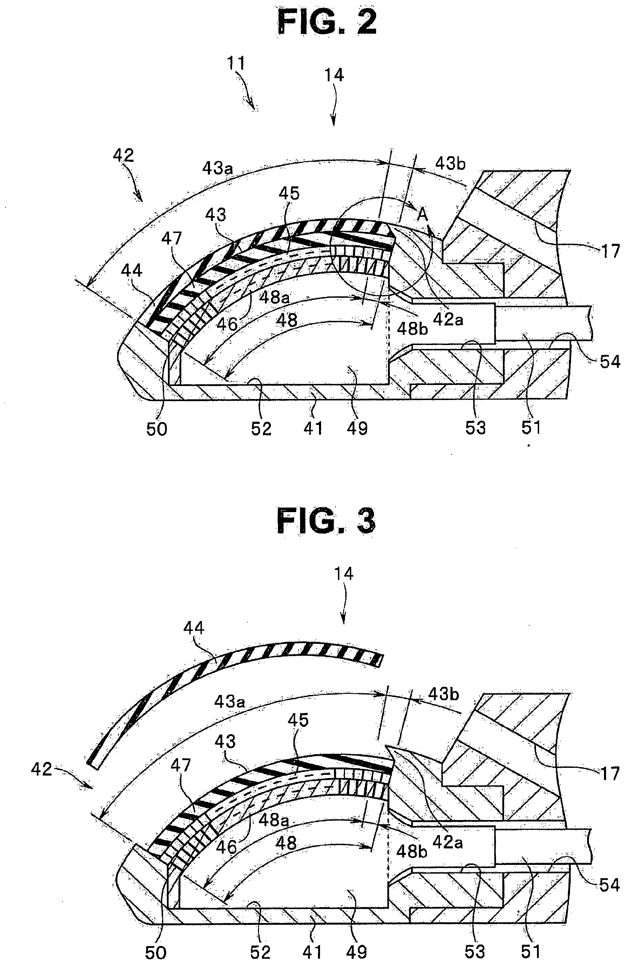

Further, in addition to an ultrasound transmitting/receiving portion 14 for transmitting/receiving ultrasound, which is described in detail later, the distal end rigid portion 11 of the insertion portion 10 is provided with an image pickup apparatus and an illumination apparatus for picking up an optical image, which are not shown, a treatment instrument insertion port 17 (see Fig. 2) for causing a treatment instrument to project, and the like.

The operation portion 20 of the ultrasound endoscope 2 is provided with an angle operation knob 15 for operating bending of the bending portion 12. Further, the operation portion 20 is provided with switches such as an air/water feeding button 16 for controlling an operation of sending out fluid from a fluid sending-out portion provided on the distal end rigid portion 11 and a suction button 18 for controlling an operation of suction from the treatment instrument insertion port 17.

The universal cord 30 of the ultrasound endoscope 2 is such that light emitted from the light source apparatus is transmitted through an optical fiber cable inserted in the universal cord 30, the operation portion 20 and the insertion portion 10 and emitted from the illumination apparatus of the distal end rigid portion 11. Note that the ultrasound endoscope 2 may be in a configuration in which the illumination apparatus arranged on the distal end rigid portion 11 is provided with the light source apparatus such as an LED. The camera control unit is configured with an apparatus configured to output an image picked up by the image pickup apparatus provided on the distal end rigid portion 11 to the monitor 4.

Further, the ultrasound observation control apparatus 3 is an apparatus configured to control an ultrasound transmitting/receiving operation by the ultrasound transmitting/receiving portion 14, generate an ultrasonogram and output the ultrasonogram to the monitor 4. Note that the ultrasound observation apparatus 1 may be configured without the ultrasound observation control apparatus 3 and the monitor 4.

Next, a configuration of a part where the ultrasound transmitting/receiving portion 14 of the ultrasound observation apparatus 1 is arranged will be described. The ultrasound transmitting/receiving portion 14 is contained and held in a housing 41 and fixed to the distal end rigid portion 11 of the insertion portion 10 via the housing 41.

An opening portion 42 is formed on the housing 41, and the ultrasound transmitting/receiving portion 14 is configured so as to transmit/receive ultrasound in an ultrasound transmitting/receiving area 43a which is a predetermined area in an ultrasound transmitting/receiving face 43 of the opening portion 42.

A proximal end side area on an outer side of the ultrasound transmitting/receiving area 43a is an ultrasound non-transmitting/receiving area 43b. That is, an outer circumferential portion 42a on a proximal end side of the opening portion 42 of the housing 41 is extended in an inclined shape interfering with an angle of a direction in which ultrasound transducers of an ultrasound transducer group 48 constituting an electroacoustic conversion portion 45 to be described later vibrate. Therefore, ultrasound transmission/reception is not performed on a part of the ultrasound transmitting/receiving face 43 overlapping with the outer circumferential portion 42a of the opening portion 42 (the ultrasound non-transmitting/receiving area 43b), and the part is an area where transmitted ultrasound beam is reflected in the housing 41.

The ultrasound transmitting/receiving face 43 is a part of a surface of the ultrasound transmitting/receiving portion 14. Though a shape of the ultrasound transmitting/receiving face 43 is not especially limited, the ultrasound transmitting/receiving portion 14 is, in the present embodiment, configured having the ultrasound transmitting/receiving face 43 constituted by a curved face forming an outward convex shape as shown in Fig. 2 as an example.

Fig. 3 shows a state that an acoustic lens portion 44 to be described in detail later, which is to be arranged on the ultrasound transmitting/receiving face 43, is removed in order to cause the ultrasound transmitting/receiving area 43a and the ultrasound non-transmitting/receiving area 43b of the ultrasound transmitting/receiving face 43 to be easily recognized.

Note that, as for directions along a normal line of the ultrasound transmitting/receiving face 43, a direction the ultrasound transmitting/receiving face 43 faces will be referred to as an outer side, and a direction opposite to the direction the ultrasound transmitting/receiving face 43 faces will be referred to as an inner side in the description below.

More specifically, the ultrasound transmitting/receiving face 43 has a shape of a substantially cylindrical face. The ultrasound transmitting/receiving face 43 is an area within a predetermined angle range in a circumferential direction in the ultrasound transmitting/receiving face 43 in the substantially cylindrical face shape, and the ultrasound non-transmitting/receiving area 43b is an area which continues from an outer circumferential end face of the opening portion 42 to the ultrasound transmitting/receiving area 43a.

The ultrasound transmitting/receiving portion 14 is capable of transmitting/receiving ultrasound beam in the direction along the normal line of the ultrasound transmitting/receiving face 43 (a diameter direction) within the ultrasound transmitting/receiving area 43a and configured so that ultrasound beam scanning can be performed in the circumferential direction of the ultrasound transmitting/receiving face 43. Further, in the ultrasound non-transmitting/receiving area 43b, transmitted ultrasound beam is reflected in the housing 41. That is, the ultrasound transmitting/receiving portion 14 of the present embodiment has a configuration in which ultrasound beam scanning is performed in a substantially arc shape. Such an ultrasound beam scanning form is generally referred to as electronic convex scanning. Note that the shape of the ultrasound transmitting/receiving face 43 may be such that is constituted by a plurality of curved faces having different curvatures or may be constituted by one or more planes. Further, the form of ultrasound beam scanning by the ultrasound transmitting/receiving portion 14 is not limited to the present embodiment.

The ultrasound transmitting/receiving portion 14 is configured having the electroacoustic conversion portion 45, backing material 46 and an acoustic matching layer 47.

The electroacoustic conversion portion 45 has a configuration for mutually converting an electrical signal and ultrasound. Though the configuration of the electroacoustic conversion portion 45 is not especially limited if it is possible to mutually convert an electrical signal and ultrasound, for example, a piezoelectric element or an electrostrictive element made of piezoelectric ceramics or the like, or a micromachined ultrasonic transducer (MUT) can be applied.

In the present embodiment, the electroacoustic conversion portion 45 is configured with the ultrasound transducer group 48 constituted by a plurality of piezoelectric elements (ultrasound transducers) arrayed in a substantially arc shape along the ultrasound transmitting/receiving face 43 on a more inner side of the ultrasound transmitting/receiving face 43, as an example.

Each of the ultrasound transducers constituting the ultrasound transducer group 48 is arranged so as to vibrate in the normal line direction (the diameter direction) of the ultrasound transmitting/receiving face 43. Further, each of the ultrasound transducers constituting the ultrasound transducer group 48 is arranged within a substantially same range as the ultrasound transmitting/receiving face 43 on a more inner side of the ultrasound transmitting/receiving face 43.

Each of all the ultrasound transducers constituting the ultrasound transducer group 48 is connected to a circuit board 49 via electrical wiring 50. Driving wiring is collected to a cable unit 51 via the circuit board 49, inserted through the ultrasound cable 33 and connected to the connector 33a. Therefore, all the ultrasound transducers constituting the ultrasound transducer group 48 can transmit/receive ultrasound beam.

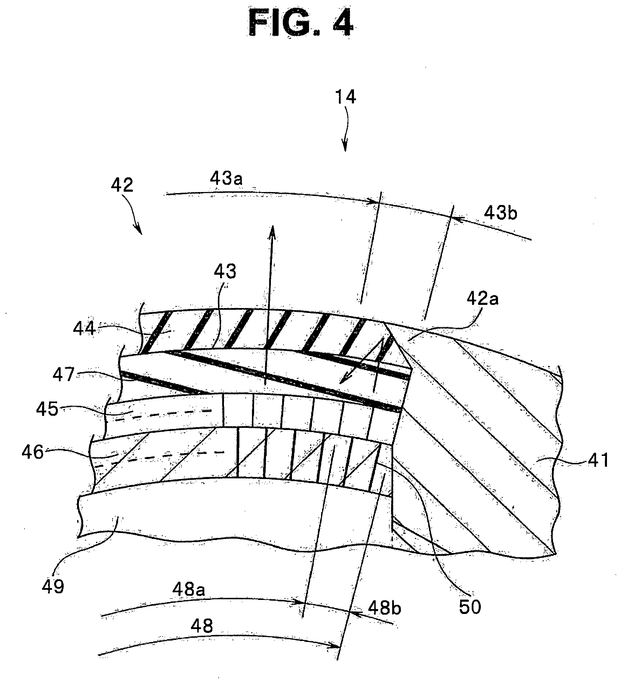

As shown in Fig. 4, the ultrasound transducer group 48 is configured with a first ultrasound transducer group 48a positioned on a back side of the ultrasound transmitting/receiving area 43a of the ultrasound transmitting/receiving face 43 and configured so that an ultrasound beam transmission wave is emitted from the opening portion 42 of the housing 41, and a second ultrasound transducer group 48b positioned on a back side of the ultrasound non-transmitting/receiving area 43b of the ultrasound transmitting/receiving face 43 and configured so that an ultrasound beam transmission wave is reflected in the housing 41.

As described above, the backing material 46 is arranged on an inner side of the electroacoustic conversion portion 45 configured with the ultrasound transducer group 48. The backing material 46 is a member configured to absorb ultrasound radiated from the electroacoustic conversion portion 45 toward the inner side and ultrasound from the inner side toward the electroacoustic conversion portion 45. Therefore, in the present embodiment, transmission/reception of ultrasound by the electroacoustic conversion portion 45 is performed only toward an outer side of the normal line direction of the ultrasound transmitting/receiving face 43.

The acoustic matching layer 47 is a member in a substantially plate shape arranged on an outer side of the electroacoustic conversion portion 45 and is configured to transmit ultrasound transmitted/received by the electroacoustic conversion portion 45. The acoustic matching layer 47 is for reducing a difference between acoustic impedances of the electroacoustic conversion portion 45 and the acoustic lens portion 44 to be described later. Note that the acoustic matching layer 47 may be in a configuration in which a plurality of layers having different densities are laminated.

The acoustic matching layer 47 is arranged so as to cover at least the whole outer side of the electroacoustic conversion portion 45. The acoustic matching layer 47 is a member arranged on a most outer side of the ultrasound transmitting/receiving portion 14 of the present embodiment. That is, the ultrasound transmitting/receiving face 43 of the ultrasound transmitting/receiving portion 14 is a main face of the outer side of the acoustic matching layer 47. Further, the ultrasound transmitting/receiving area 43a is an area where ultrasound transmitted/received by the electroacoustic conversion portion 45 passes through within the face on the outer side of the acoustic matching layer 47.

On the ultrasound transmitting/receiving face 43 of the ultrasound transmitting/receiving portion 14 having the configuration as described above, the acoustic lens portion 44 is arranged. The acoustic lens portion 44 is for causing ultrasound beam transmitted by the ultrasound transmitting/receiving portion 14 to converge.

Material constituting the acoustic lens portion 44 is not especially limited and is determined in consideration of a density appropriate for acoustic impedance matching with a subject, chemical resistance and the like. In the present embodiment, since a subject is a living body such as a human body, the acoustic lens portion 44 is configured with silicone resin as an example.

Note that a shape of a section of the acoustic lens portion 44 is determined according to a density of material constituting the acoustic lens portion 44, a wavelength of ultrasound the ultrasound transmitting/receiving portion 14 transmits, a width of the ultrasound transmitting/receiving face 43, a distance for convergence, and the like and is not especially limited.

When seen from the outer side of the normal line direction of the ultrasound transmitting/receiving face 43, the acoustic lens portion 44 has such an external form that covers at least the entire ultrasound transmitting/receiving area 43a. In the present embodiment, when seen from the outer side of the normal line direction of the ultrasound transmitting/receiving face 43, the acoustic lens portion 44 extends over a circumference of the ultrasound transmitting/receiving area 43a, and the ultrasound transmitting/receiving face 43 extends over a circumference of the acoustic lens portion 44, as an example. Note that, in the present embodiment, when seen from the outer side of the normal line direction of the ultrasound transmitting/receiving face 43, a distal end side of the acoustic lens portion 44 is formed, substantially corresponding to the ultrasound transmitting/receiving area 43a. The acoustic lens portion 44 is fixed on the ultrasound transmitting/receiving face 43, which is an outer surface of the acoustic matching layer 47, for example, with silicone-based adhesive.

The ultrasound transmitting/receiving portion 14, the acoustic lens portion 44 and the like having the configurations described above are contained and held in a recess portion 52 of the housing 41, and the housing 41 is fixed to the distal end rigid portion 11. The driving wiring from the circuit board 49 contained in the recess portion 52 of the housing 41 is collected to the cable unit 51, passes through the housing 41, passes through a hole 53 opened in the recess portion 52 and the distal end rigid portion 11, is inserted inside a hole 54 causing the bending portion 12 and the hole 53 to communicate with each other, is inserted in the ultrasound cable 33 and is connected to the connector 33a.

Thus, according to the first embodiment of the present invention, a configuration is adopted in which each of all the ultrasound transducers constituting the ultrasound transducer group 48 is connected to the circuit board 49 via the electrical wiring 50 so that all the ultrasound transducers can transmit/receive ultrasound. Therefore, even if the second ultrasound transducer group 48b which causes ultrasound to be reflected in the housing 41 occurs due to a shape (inclination and an amount of projection), influence and the like of the outer circumferential portion 42a of the opening portion 42 of the housing 41, the first ultrasound transducer group 48a capable of emitting ultrasound from the opening portion 42 can be appropriately secured. Therefore, careful positioning, formation of a fitting portion or the like in consideration of the shape (inclination and the amount of projection), the influence and the like of the outer circumferential portion 42a of the opening portion 42 of the housing 41 are not required. Therefore, it is possible to easily and accurately form the ultrasound transmitting/receiving portion 14, and it becomes possible to easily realize downsizing, high resolution and high functionality of an ultrasound endoscope.

Note that, though description has been made on the example in which the second ultrasound transducer group 48b which causes ultrasound to be reflected in the housing 41 is positioned on the proximal end side of the opening portion 42 of the housing 41 in the present first embodiment, the second ultrasound transducer group 48b may be positioned on a distal end side of the opening portion 42 of the housing 41 or may be positioned on both of the distal end side and the proximal end side.

### (Second Embodiment)

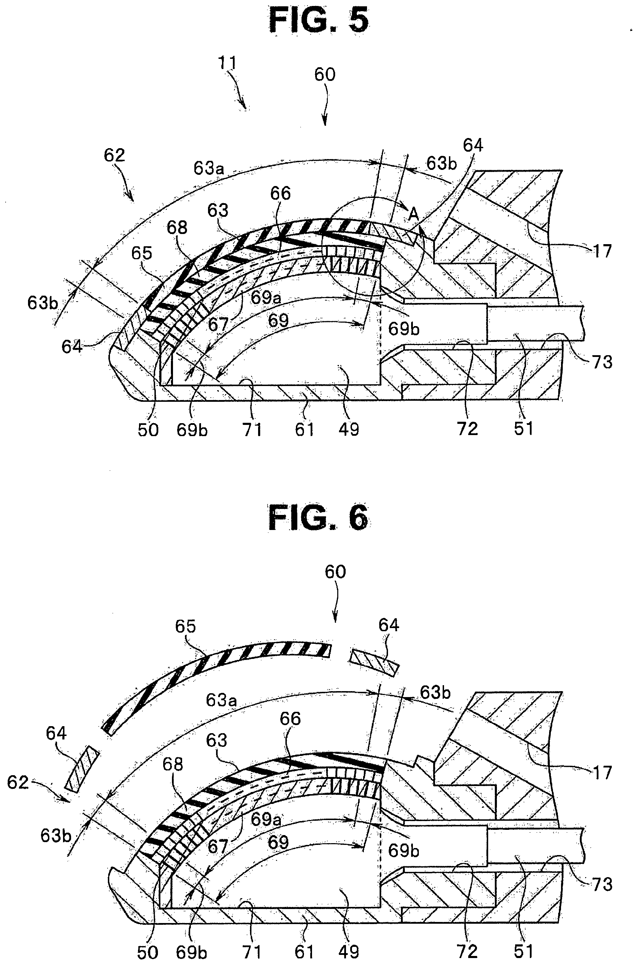

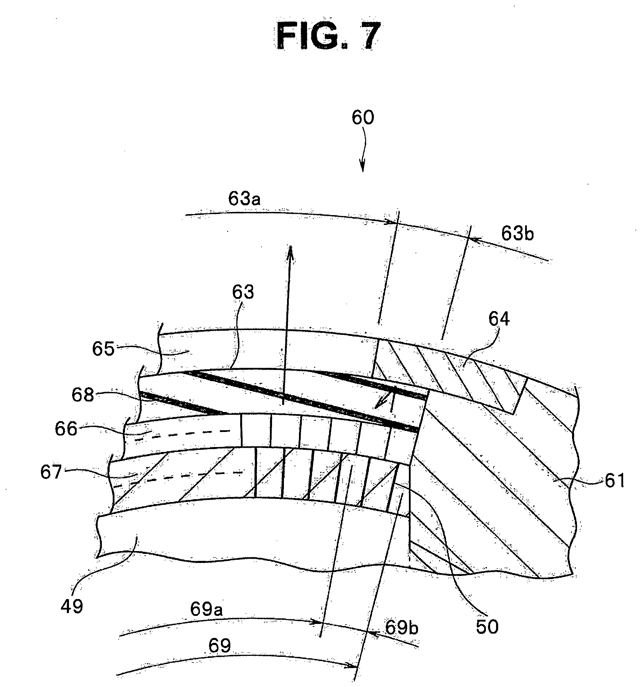

Next, Figs. 5 to 7 show a second embodiment. Note that the second embodiment is different from the first embodiment in that a configuration is adopted in which protecting portions are provided on an outer circumferential portion of the housing of the ultrasound transmitting/receiving portion of the ultrasound endoscope of the first embodiment, and the second ultrasound transducer group is set by the protecting portion. Since other components are the same as the components in the first embodiment, the same components will be given the same reference numerals, and description of the components will be omitted.

A configuration of a part where an ultrasound transmitting/receiving portion 60 according to the second embodiment is arranged will be described. The ultrasound transmitting/receiving portion 60 is contained and held in a housing 61 and fixed to the distal end rigid portion 11 via the housing 61.

An opening portion 62 is formed on the housing 61, and the ultrasound transmitting/receiving portion 60 is configured so as to transmit/receive ultrasound in an ultrasound transmitting/receiving area 63a which is a predetermined area in an ultrasound transmitting/receiving face 63 of the opening portion 62.

A distal end side area and a proximal end side area on an outer side of the ultrasound transmitting/receiving area 63a are ultrasound non-transmitting/receiving areas 63b as described later. That is, the distal end side area and the proximal end side area on the outer side of the ultrasound transmitting/receiving area 63 a are covered with protecting portions 64, respectively, as described in detail later. Therefore, ultrasound transmission/reception is not performed on parts of the ultrasound transmitting/receiving face 63 overlapping with the protecting portions 64 (the ultrasound non-transmitting/receiving areas 63b), and the parts are areas where transmitted ultrasound beam is reflected in the housing 61.

The ultrasound transmitting/receiving face 63 is a part of a surface of the ultrasound transmitting/receiving portion 60. Though a shape of the ultrasound transmitting/receiving face 63 is not especially limited, the ultrasound transmitting/receiving portion 60 is, in the present embodiment, configured having the ultrasound transmitting/receiving face 63 constituted by a curved face forming an outward convex shape as shown in Fig. 5 as an example, similarly to the first embodiment.

Fig. 6 shows a state that an acoustic lens portion 65 and the protecting portions 64 to be described in detail later, which are arranged on the ultrasound transmitting/receiving face 63, are removed in order to cause the ultrasound transmitting/receiving area 63a and the ultrasound non-transmitting/receiving areas 63b of the ultrasound transmitting/receiving face 63 to be easily recognized.

Note that, as for a direction along a normal line of the ultrasound transmitting/receiving face 63, a direction the ultrasound transmitting/receiving face 63 faces will be referred to as an outer side, and a direction opposite to the direction the ultrasound transmitting/receiving face 63 faces will be referred to as an inner side in the description below.

More specifically, the ultrasound transmitting/receiving face 63 has a shape of a substantially cylindrical face. The ultrasound transmitting/receiving area 63a is an area within a predetermined angle range in a circumferential direction in the ultrasound transmitting/receiving face 63 in the substantially cylindrical face shape. The ultrasound transmitting/receiving area 63a is smaller than the ultrasound transmitting/receiving face 63 and formed at a center of the ultrasound transmitting/receiving face 63. On both end sides of the ultrasound transmitting/receiving area 63a, the ultrasound non-transmitting/receiving areas 63b are formed.

The ultrasound transmitting/receiving portion 60 is capable of transmitting ultrasound beam in the direction along the normal line of the ultrasound transmitting/receiving face 63 (a diameter direction) within the ultrasound transmitting/receiving area 63a and configured so that ultrasound beam scanning can be performed in the circumferential direction of the ultrasound transmitting/receiving face 63, and the ultrasound transmitting/receiving portion 60 is such that electronic convex scanning similar to that of the first embodiment is performed. Note that the shape of the ultrasound transmitting/receiving face 63 may be such that is constituted by a plurality of curved faces having different curvatures or may be constituted by one or more planes. Further, the form of ultrasound beam scanning by the ultrasound transmitting/receiving portion 60 is not limited to that of the present embodiment.

The ultrasound transmitting/receiving portion 60 is configured having an electroacoustic conversion portion 66, backing material 67 and an acoustic matching layer 68.

The electroacoustic conversion portion 66 is similar to the electroacoustic conversion portion of the first embodiment and is configured with an ultrasound transducer group 69 constituted by a plurality of piezoelectric elements (ultrasound transducers) arrayed in a substantially arc shape along the ultrasound transmitting/receiving face 63 on a more inner side of the ultrasound transmitting/receiving face 63.

Each of the ultrasound transducers constituting the ultrasound transducer group 69 is arranged so as to vibrate in the normal line direction (the diameter direction) of the ultrasound transmitting/receiving face 63. Further, each of the ultrasound transducers constituting the ultrasound transducer group 69 is arranged within a substantially same range as the ultrasound transmitting/receiving face 63 on a more inner side of the ultrasound transmitting/receiving face 63.

Each of all the ultrasound transducers constituting the ultrasound transducer group 69 is connected to the circuit board 49 via the electrical wiring 50. Driving wiring is collected to the cable unit 51 via the circuit board 49, inserted through the ultrasound cable 33 and connected to the connector 33a. Therefore, all the ultrasound transducers constituting the ultrasound transducer group 69 can transmit/receive ultrasound beam.

The ultrasound transducer group 69 is configured with a first ultrasound transducer group 69a positioned on a back side of the ultrasound transmitting/receiving area 63a of the ultrasound transmitting/receiving face 63 and configured so that an ultrasound beam transmission wave is emitted from the opening portion 62 of the housing 61, and a second ultrasound transducer group 69b positioned on the back side of the ultrasound non-transmitting/receiving areas 63b of the ultrasound transmitting/receiving face 63, which are covered with the protecting portions 64, and configured so that an ultrasound beam transmission wave is reflected in the housing 61.

As described above, the backing material 67 is arranged on an inner side of the electroacoustic conversion portion 66 configured with the ultrasound transducer group 69. Since the backing material 67 is similar to the backing material of the first embodiment, description will be omitted. Further, since the acoustic matching layer 68 provided so as to cover the electroacoustic conversion portion 66 is also similar to the acoustic matching layer of the first embodiment, description will be omitted.

On the ultrasound transmitting/receiving face 63 of the ultrasound transmitting/receiving portion 60 having the configuration as described above, the acoustic lens portion 65 configured to cause ultrasound beam to converge, which is similar to the acoustic lens portion of the first embodiment, is arranged, and both end portions of the acoustic matching layer 68 are extended in an outer circumferential direction more than both end portions of acoustic lens portion 65.

The acoustic lens portion 65 is fixed on the ultrasound transmitting/receiving face 63, which is an outer surface of the acoustic matching layer 68 with first adhesive. Though a kind of the first adhesive is not especially limited, for example, silicone-based adhesive is used.

A pair of protecting portions 64 is arranged on outer side faces of both end portions of the acoustic matching layer 68. Further, the pair of protecting portions 64 is in contact with both end portions of the acoustic lens portion 65, and extends toward a more outer side than the end portion of the acoustic matching layer 68 when seen from the outer side of the normal line direction of the ultrasound transmitting/receiving face 63. That is, the protecting portions 64 are arranged so as to cover an outer circumferential portion, which is an area not covered with the acoustic lens portion 65 of the ultrasound transmitting/receiving face 63, and extend in an outer circumferential direction more than the ultrasound transmitting/receiving face 63.

The protecting portions 64 are bonded to both of an outer circumferential portion of the acoustic matching layer 68 and the end portions of the acoustic lens portion 65 with second adhesive. Here, kinds of material constituting the protecting portions 64 and the second adhesive are selected so that adhesion strength per unit area between the acoustic matching layer 68 and the protecting portions 64 by the second adhesive is higher than adhesion strength per unit area between the acoustic matching layer 68 and the acoustic lens portion 65 by the first adhesive.

In the present embodiment, the acoustic lens portion 65 is made of silicone resin, and silicone-based adhesive is used as the first adhesive for bonding the acoustic matching layer 68 and the acoustic lens portion 65 to each other as an example. Therefore, if the protecting portions 64 are configured with so-called engineering plastic, for example, polyimide, polyamide or polysulphone, and epoxy-based adhesive is used as the second adhesive, the adhesion strength per unit area between the acoustic matching layer 68 and the protecting portions 64 by the second adhesive is higher than the adhesion strength per unit area between the acoustic lens portion 65 and the acoustic matching layer 68 by the first adhesive.

Further, affinity of surfaces of the end portions of the acoustic lens portion 65 to be bonded to the protecting portions 64 for the second adhesive has been increased by chemical treatment. Here, though a method for the chemical treatment for increasing the adhesion affinity between the acoustic lens portion 65 and the second adhesive is not especially limited, well-known techniques, for example, plasma treatment, ion beam treatment or treatment by acid can be applied.

The silicone resin constituting the acoustic lens portion 65 is generally a member with a low affinity for adhesive and a relatively low adhesion strength. However, by performing treatment for increasing the adhesion affinity between the acoustic lens portion 65 and the second adhesive, adhesion strength between the acoustic lens portion 65 and the protecting portions 64 can be increased, and detachment between the acoustic lens portion 65 and the protecting portions 64 can be prevented.

The ultrasound transmitting/receiving portion 60, the acoustic lens portion 65, the protecting portions 64 and the like having the configurations described above are contained and held in a recess portion 71 of the housing 61, and the housing 61 is fixed to the distal end rigid portion 11. The driving wiring from the circuit board 49 contained in the recess portion 71 of the housing 61 is collected to the cable unit 51, passes through the housing 61, passes through a hole 72 opened in the recess portion 71 and the distal end rigid portion 11, is inserted inside a hole 73 causing the bending portion 12 and the hole 72 to communicate with each other, is inserted in the ultrasound cable 33 and connected to the connector 33a.

As described above, the ultrasound endoscope 2 according to the present second embodiment includes the ultrasound transmitting/receiving portion 60 configured to transmit/receive ultrasound on the ultrasound transmitting/receiving face 63, the housing 61 configured to hold the ultrasound transmitting/receiving portion 60 so that the ultrasound transmitting/receiving face 63 is exposed, and the acoustic lens portion 65 bonded on the ultrasound transmitting/receiving face 63 so as to cover the ultrasound transmitting/receiving area 63a. The ultrasound endoscope 2 is configured, further having the protecting portions 64 configured to cover the outer circumferential portion of the ultrasound transmitting/receiving face 63 which is not covered with the acoustic lens portion 65, arranged so as to extend in the outer circumferential direction more than the ultrasound transmitting/receiving face 63, and bonded on the acoustic lens portion 65, the outer circumferential portion of the ultrasound transmitting/receiving face 63 and the housing 61. The protecting portions 64 are bonded from the outer circumferential portion of the ultrasound transmitting/receiving face 63 to the recess portion 71 of the housing 61 with adhesive.

In the ultrasound endoscope 2 of the present embodiment having such a configuration, since the protecting members 64 bonded to both of the acoustic matching layer 68 and the housing 61 are arranged on an outer side of a boundary portion between the acoustic matching layer 68 and the housing 61, a route for liquid to enter the housing 61 is lengthened, and, thereby, waterproofness of the transducers is improved more.

Further, for example, even if unexpected excessive external force is applied to the acoustic lens portion 65, and lens holding force by adhesive between the acoustic matching layer 68 and the protecting portions 64 decreases, watertightness inside the housing 61 is kept by adhesion between the protecting portions 64 and the acoustic matching layer 68/the housing 61, and, therefore, it is possible to prevent liquid from entering the housing 61.

Further, adhesion strength per unit area between the protecting portions 64 and the acoustic matching layer 68/the housing 61 by the second adhesive is higher than the adhesion strength per unit area between the acoustic lens portion 65 and the acoustic matching layer 68 by the first adhesive. Therefore, even if such unexpected excessive external force that the acoustic lens portion 65 is detached from the acoustic matching layer 68 is applied, detachment of the protecting portions 64 from the acoustic matching layer 68 and the housing 61 can be suppressed.

Further, in the ultrasound endoscope 2 according to the second embodiment, the ultrasound transmitting/receiving area 63a of the ultrasound transmitting/receiving face 63 is set between (a proximal end face of) a distal end side protecting portion 64 and (a distal end face of) a proximal end side protecting portion 64. Therefore, in order to form the ultrasound transmitting/receiving area 63a on the electroacoustic conversion portion 66 from beginning and perform assembly so that the ultrasound transmitting/receiving area 63a overlaps with an ultrasound transmitting/receiving area 63 a set by the pair of protecting portions 64 described above, careful positioning in consideration of the shape (inclination, the amount of projection),influence and the like of the pair of protecting portions 64, formation of a fitting portion and the like are required. According to the second embodiment of the present invention, however, since a configuration is adopted in which each of all the ultrasound transducers constituting the ultrasound transducer group 69 is connected to the circuit board 49 via the electrical wiring 50 so that all the ultrasound transducers can transmit/receive ultrasound, it is possible to appropriately secure the first ultrasound transducer group 69a capable of emitting ultrasound from the opening portion 62 even if the second ultrasound transducer group 69b which causes ultrasound to be reflected in the housing 41 occur due to the shape (inclination and the amount of projection), influence and the like of the pair of protecting portions 64. Therefore, it is not necessary to perform careful positioning, formation of a fitting portion and the like in consideration of the shape (inclination and the amount of projection), influence and the like of the pair of protecting portions 64; it is possible to easily and accurately form the ultrasound transmitting/receiving portion 60; and it becomes possible to easily realize downsizing, high resolution and high functionality of an ultrasound endoscope.

### (Third Embodiment)

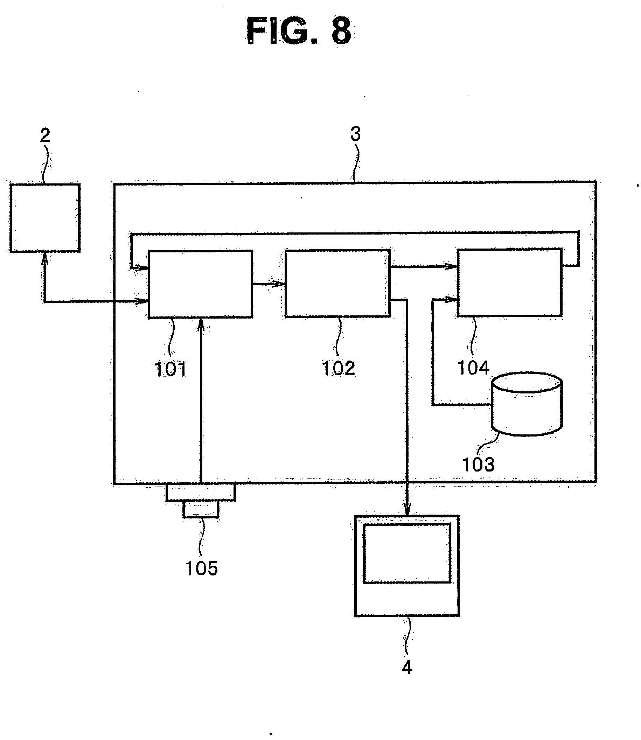

Next, Fig. 8 is a functional block diagram of an ultrasound observation control apparatus according to a third embodiment of the present invention. Note that the present third embodiment is such that the first ultrasound observation control apparatus receives a signal from the ultrasound transmitting/receiving portion of the ultrasound endoscope according to the first embodiment, selects a first ultrasound transducer group and drives only the first ultrasound transducer group. Since other components and operations are similar to those of the first embodiment, the same components will be given the same reference numerals, and description of the components will be omitted.

That is, as shown in Fig. 8, an ultrasound observation control portion 3 is configured mainly being provided with a transmission/reception control portion 101, a B-mode image calculating portion 102, a storage portion 103, a correlation calculating portion 104 and an ultrasound observation switch 105. Note that implementation of the transmission/reception control portion 101, the B-mode image calculating portion 102 and the correlation calculating portion 104 to the ultrasound observation control portion 3 may be by hardware or by software.

The transmission/reception control portion 101 controls transmission/reception of ultrasound beam by the ultrasound transmitting/receiving portion 14 of the ultrasound endoscope 2. That is, control of a position on a scan plane and control of ultrasound beam scanning for obtaining a B-mode image on the scan plane are performed by the transmission/reception control portion 101.

More specifically, in the ultrasound transmitting/receiving area 43a in the ultrasound transmitting/receiving face 43 of the ultrasound transmitting/receiving portion 14, ultrasound beam from the first ultrasound transducer group 48a of the ultrasound transducer group 48 constituting the electroacoustic conversion portion 45 is emitted from the opening portion 42 of the housing 41 and received, and an appropriate ultrasound image can be obtained. On the other hand, in the ultrasound non-transmitting/receiving area 43b in the ultrasound transmitting/receiving face 43, ultrasound beam transmitted from the second ultrasound transducer group 48b of the ultrasound transducer group 48 is reflected in the housing 41, and a situation that an ultrasound image cannot be obtained occurs.

Then, when detecting the situation, the transmission/reception control portion 101 judges a group of ultrasound transducers arranged on a part where an appropriate ultrasound image can be obtained to be the first ultrasound transducer group 48a, and a group of the other ultrasound transducers to be the second ultrasound transducer group 48b, and performs control so that only the first ultrasound transducer group is driven.

Thereby, a group of minimum ultrasound transducers to obtain an ultrasound image, that is, only the first ultrasound transducer group 48a is driven, and control is performed so that image observation with little loss in driving power, a calorific value and the like can be performed. Note that the transmission/reception control portion 101 may drive not all the ultrasound transducers of the first ultrasound transducer group 48a corresponding to the ultrasound transmitting/receiving area 43a but a part of the first ultrasound transducer group 48a.

The B-mode image calculating portion 102 generates a B-mode image on the scan plane from a result of ultrasound beam scanning by the ultrasound transmitting/receiving portion 14 of the ultrasound endoscope 2. For example, if a treatment instrument (not shown) exists on the scan plane, an echo pattern of the treatment instrument appears in the B-mode image.

The storage portion 103 stores predetermined sample images specified according to shapes of treatment instruments. More specifically, a sample image is an image indicating a shape and size of an idealistic echo pattern of a treatment instrument in a B-mode image when the scan plane and a central axis of the treatment instrument correspond to each other.

For example, if the treatment instrument is a puncture needle, the sample image is an image indicating a shape and size of an echo pattern of a distal end portion of the puncture needle when a central axis of the puncture needle corresponds to the scan plane. The sample images are created in advance according to kinds and shapes of treatment instruments.

The correlation calculating portion 104 calculates a correlation value R between a B-mode image and a sample image. More specifically, the correlation calculating portion 104 performs image processing called pattern matching, with a sample image as a template, and calculates a degree of similarity between an echo pattern in the B-mode image and the sample image. The higher the degree of similarity between the echo pattern in the B-mode image and the sample image is, the higher the correlation value R is. The pattern matching is a well-known technique, detailed description will be omitted.

The ultrasound observation switch 105 is an input device for a user to input instructions to start and end observation by a B-mode image. In the present embodiment, the ultrasound observation switch 105 is provided on the ultrasound observation control portion 3 as an example. However, the ultrasound observation switch 105 may be configured so as to be provided in the operation portion 20 of the ultrasound endoscope 2 or may be in a form of being provided, being separated from the ultrasound observation control portion 3 and the ultrasound endoscope 2 like a foot switch.

Thus, in the ultrasound observation apparatus 1 of the present third embodiment, it becomes possible to perform image observation with little loss in driving power, a calorific value and the like by the ultrasound observation control portion 3, in addition to the effects of the ultrasound endoscopes according to the first and second embodiments.

## Claims

1. An ultrasound endoscope (2) comprising:
a housing (41) in which an opening portion (42) is formed;
an ultrasound transmitting/receiving portion (14) comprising a first ultrasound transducer group (48a) comprising at least one ultrasound transducer and causing a transmission wave to be emitted from the opening portion and a second ultrasound transducer group (48b) comprising at least one ultrasound transducer and causing a transmission wave to be reflected in the housing; and
wiring (50) connecting to each of ultrasound transducers constituting the first ultrasound transducer group and ultrasound transducers constituting the second ultrasound transducer group.

2. The ultrasound endoscope according to claim 1, wherein
the housing comprises a protecting portion covering an outer circumference of the opening portion; and
the ultrasound endoscope comprises an outer circumferential portion of the opening portion of the housing, and the transmission wave from the second ultrasound transducer group is reflected by an inner face of the protecting portion.

3. An ultrasound observation apparatus (1) comprising:
the ultrasound endoscope according to claim 1;
a connection portion electrically connecting to the wiring of the ultrasound endoscope; and
a control portion connecting to the connection portion and configured to select the first ultrasound transducer group from the ultrasound transmitting/receiving portion of the ultrasound endoscope and drive only the first ultrasound transducer group.

4. An ultrasound endoscope according to claim 1, further comprising:
a transmission/reception control portion (101) connected to the ultrasound transmitting/receiving portion via the wiring and configured to detect reflection in the housing, determine the second ultrasound transducer group based on a detection result and perform control so as to drive the first ultrasound transducer group that is not included in the second ultrasound transducer group.

## Patentansprüche

1. Ultraschallendoskop (2), umfassend:
ein Gehäuse (41), in dem ein Öffnungsabschnitt (42) ausgebildet ist;
einen Ultraschallübertragungs-/-empfangsabschnitt (14), der eine erste Ultraschallwandlergruppe (48a), die mindestens einen Ultraschallwandler umfasst und bewirkt, dass eine Übertragungswelle von dem Öffnungsabschnitt emittiert wird, und eine zweite Ultraschallwandlergruppe (48b), die mindestens einen Ultraschallwandler umfasst und bewirkt, dass eine Übertragungswelle in dem Gehäuse reflektiert wird, umfasst; und
eine Verkabelung (50), die mit jedem der Ultraschallwandler, die die erste Ultraschallwandlergruppe bilden, und der Ultraschallwandler, die die zweite Ultraschallwandlergruppe bilden, eine Verbindung herstellt.

2. Ultraschallendoskop nach Anspruch 1, wobei
das Gehäuse einen Schutzabschnitt umfasst, der einen Außenumfang des Öffnungsabschnitts abdeckt; und
das Ultraschallendoskop einen Außenumfangsabschnitt des Öffnungsabschnitts des Gehäuses umfasst und die Übertragungswelle von der zweiten Ultraschallwandlergruppe von einer Innenfläche des Schutzabschnitts reflektiert wird.

3. Ultraschallbeobachtungsvorrichtung (1), umfassend:
das Ultraschallendoskop nach Anspruch 1;
einen Verbindungsabschnitt, der mit der Verkabelung des Ultraschallendoskops eine elektrische Verbindung herstellt; und
einen Steuerabschnitt, der mit dem Verbindungsabschnitt eine Verbindung herstellt und dazu konfiguriert ist, die erste Ultraschallwandlergruppe von dem Ultraschallübertragungs-/-empfangsabschnitt des Ultraschallendoskops auszuwählen und nur die erste Ultraschallwandlergruppe anzutreiben.

4. Ultraschallendoskop nach Anspruch 1, weiterhin umfassend:
einen Übertragungs-/Empfangssteuerabschnitt (101), der mittels der Verkabelung mit dem Ultraschallübertragungs-/-empfangsabschnitt eine verbunden und dazu konfiguriert ist, eine Reflexion in dem Gehäuse zu erfassen, die zweite Ultraschallwandlergruppe auf der Basis eines Erfassungsergebnisses zu bestimmen und eine Steuerung durchzuführen, um die erste Ultraschallwandlergruppe, die nicht von der zweiten Ultraschallwandlergruppe umfasst wird, anzutreiben.

## Revendications

1. Endoscope à ultrasons (2) comprenant :
un boîtier (41) dans lequel une partie d'ouverture (42) est formée ;
une partie d'émission/réception d'ultrasons (14) comprenant un premier groupe transducteur à ultrasons (48a) comprenant au moins un transducteur à ultrasons et amenant une onde d'émission à être émise à partir de la partie d'ouverture et un second groupe transducteur à ultrasons (48b) comprenant au moins un transducteur à ultrasons et amenant une onde d'émission à être réfléchie dans le boîtier ; et
un câblage (50) reliant chacun des transducteurs à ultrasons constituant le premier groupe transducteur à ultrasons et des transducteurs à ultrasons constituant le second groupe transducteur à ultrasons.

2. Endoscope à ultrasons selon la revendication 1, dans lequel
le boîtier comprend une partie de protection recouvrant une circonférence externe de la partie d'ouverture ; et
l'endoscope à ultrasons comprend une partie circonférentielle externe de la partie d'ouverture du boîtier, et l'onde d'émission provenant du second groupe transducteur à ultrasons est réfléchie par une face interne de la partie de protection.

3. Appareil d'observation à ultrasons (1) comprenant :
l'endoscope à ultrasons selon la revendication 1 ;
une partie de liaison se reliant électriquement au câblage de l'endoscope à ultrasons ; et
une partie de commande se reliant à la partie de liaison et configurée pour sélectionner le premier groupe transducteur à ultrasons à partir de la partie d'émission/réception d'ultrasons de l'endoscope à ultrasons et piloter uniquement le premier groupe transducteur à ultrasons.

4. Endoscope à ultrasons selon la revendication 1, comprenant en outre :
une partie de commande d'émission/réception (101) reliée à la partie d'émission/réception d'ultrasons par l'intermédiaire du câblage et configurée pour détecter une réflexion dans le boîtier, déterminer le second groupe transducteur à ultrasons sur la base d'un résultat de détection, et réaliser une commande de façon à piloter le premier groupe transducteur à ultrasons qui n'est pas inclus dans le second groupe transducteur à ultrasons.
